# EUROPÄISCHE PATENTSCHRIFT

(11) **EP 1 302 193 B1**
(45) Veröffentlichungstag und Bekanntmachung des Hinweises auf die Patenterteilung: **08.12.2004**
(21) Anmeldenummer: 03001081.3
(22) Anmeldetag: 18.01.2003
(51) Int. Cl.: A61K 7/06

(54) **Haarwachsprodukt aus Polyethylenglykolwachsen und hydrophoben Stoffen**
Waxy hair composition containing poly(ethylene glycol) waxes and hydrophobic compounds
Composition capillaire cireuse contenant des cires de poly(éthylène glycol) et des matières hydrophobes

(30) Priorität: 09.11.2002 DE 10252167
(43) Veröffentlichungstag der Anmeldung: 16.04.2003
(73) Patentinhaber: Wella Aktiengesellschaft, 64274 Darmstadt (DE)
(72) Erfinder: Hannich, Manuela, 63329 Egelsbach (DE); Franzke, Michael, 64380 Rossdorf (DE)

(56) Entgegenhaltungen:
- DE-C- 10 047 038
- GB-A- 1 563 824
- US-A- 3 341 410
- US-A- 4 296 763
- US-A- 5 690 924
- D.F. WILLIAMS AND W.F. SCHMITT, ED.: "Chemistry and technology of the cosmetics and toiletries industry, 2nd ed., 1996" , BLACKIE ACADEMIC & PROFESSIONAL XP002237428 229190 * Seite 83 *

## Beschreibung

Gegenstand der Erfindung ist ein Wachsprodukt zur Behandlung oder Erstellung der menschlichen Frisur. Das Wachsprodukt enthält eine Zusammensetzung mit einem Gehalt an hydrophilen, wachsartigen Polyethylenglykolen sowie hydrophoben Stoffen.

Stylingwachs-Zusammensetzungen sind bekannte Produkte zur Haarbehandlung. Sie finden insbesondere Anwendung, um kurzes bis mittellanges Haar trendgerecht in Form zu bringen und der Frisur Halt, Stand und Festigung sowie Glanz zu verleihen. Auch lassen sich mit Haarwachsen Konturen und Texture in der Frisur erzeugen. Herkömmliche Haarwachse werden üblicherweise in Tiegeln angeboten und beruhen auf folgendem Wirkungsprinzip: Die Entnahme der Produktmasse erfolgt mit den Fingern. Das Wachs wird in den Handflächen verteilt und durch die Handwärme aufgeschmolzen oder zumindest stark erweicht. Durch die Erweichung oder Aufschmelzung wird die Einarbeitung des ansonsten zu festen Wachses in das Haar ermöglicht. Im erweichten oder mehr oder weniger flüssigen Zustand wird das Wachs in das Haar eingearbeitet. Im Haar kühlt es ab und erreicht wieder seine Ausgangskonsistenz. Dabei erhärtet es und die gestaltete Frisur erhält Stabilität und Halt sowie häufig einen leichten Wet-Look. Herkömmliche Haarwachsprodukte, wie sie derzeit angeboten werden, sind in der Regel auf der Basis von hydrophoben Wachsen, Fetten und Ölen aufgebaut. Sie enthalten einen großen Anteil an hydrophoben Stoffen wie z.B. pflanzlichen oder tierischen Wachsen, Fettsäureestern, Fettalkoholen etc.. Hauptbestandteile und Hauptwirkstoffe sind hydrophobe Wachse wie z.B. Ozokerit, Candelillawachs, Bienenwachs, Carnaubawachs etc. Derartige Produkte haben den Nachteil, dass sie schlecht aus dem Haar auswaschbar sind und eine relativ hohe Belastung des Haares bewirken. Daher wurden auch Haarwachse auf der Basis von Mischungen von Polyethylenglykolwachsen (PEG'S) entwickelt, die eine gute Wasserlöslichkeit und eine gute Auswaschbarkeit zeigen. Für eine gute Produktleistung ist es erforderlich, Mischungen von PEG's verschiedener Molekulargewichte einzusetzen. Aus der EP 0 301 197 A1 ist ein Haarwachs bekannt, welches eine Kombination von hochmolekularem PEG (MG = 3000 bis 5000), oxethyliertem und hydriertem Rizinusöl und niedrigmolekularem PEG (MG = 100 bis 300) enthält. Ein Nachteil dieser Haarwachsprodukte besteht darin, dass gelegentlich eine Kristallisierung oder eine partielle Kristallisierung der hochmolekularen PEG's auftreten kann, die der Masse ein unhomogenes, körniges Aussehen verleiht. Bei den in der Regel in erwärmtem, fließfähigen Zustand abgefüllten Produktmassen kann es zu Inhomogenitäten der Wachsmasse nach dem Abkühlen kommen, was sich dadurch bemerkbar macht, dass die Wachsmasse im Inneren der Verpackung weicher ist als in den Außenbereichen.

In der GB 1 563 824 werden Haarwuchsmittel beschrieben. Beispiel 3 ist eine Haarwuchscreme, welche u.a. Polyethylenglykol 4000 und Vaseline enthält. In der US 3,341,410 werden therapeutische Hautbehandlungsmittel und Verfahren zur Unterstützung der Epithelisierung der Haut. Beispiel III ist eine therapeutische Zusammensetzung, welche u.a. Carbowax 1540 und Para-Wax enthält. Beispiel VI ist eine Zusammensetzung zur Behandlung von Infektionen, welche u.a. Carbowax 4000 und Erdnußöl enthält. Die US 4,296,763 beschreibt ein Verfahren zum Aufbringen eines haarkonditionierenden heißen Öls auf Haare. Hierzu wird eine auf den Kopfapplizierbare Kappe aus einem porösem Polymerschaum mit einer das Haar revitalisierenden Ölzusammensetzung imprägniert. Die Ölzusammensetzung kann u.a. Polyglykolwachs, Olivenöl und Lanolin enthalten.

Die Aufgabe bestand darin, ein Haarwachsprodukt zur Verfügung zu stellen, welches sowohl eine gute Auswaschbarkeit zeigt als auch weniger zu partieller Kristallisierung neigt, die Masse also auch über einen längeren Zeitraum homogener und weniger körnig aussieht. Darüberhinaus soll das Produkt gut dem Verpackungsmittel entnehmbar und gut verarbeitbar sein und gute Anwendungseigenschaften auf dem Haar aufweisen, insbesondere gute Einarbeitbarkeit, gute Verteilbarkeit und gute Frisurendefinition und der Frisur Glanz, Halt, Festigung bzw. Stand verleihen.

Es wurde nun gefunden, dass die Anforderungen erfüllt werden durch ein Haarwachsprodukt zur Behandlung oder Erstellung der menschlichen Frisur, welches neben hydrophilen Polyethylenglykolwachsen einen Zusatz eines hydrophoben, die Kristallisation des Polyethylenglykolwachses in der Zusammensetzung verhindernden Stoffes enthält. Gegenstand der Erfindung ist daher ein Haarwachsprodukt mit wachsartiger Konsistenz zur Behandlung oder Erstellung der menschlichen Frisur mit einem Gehalt an
(A)25 bis 60 Gew.% mindestens eines bei Raumtemperatur wachsartig festen Polyethylenglykols und
(B) größer 5 bis 30 Gew.% mindestens eines die Kristallisation des Polyethylenglykols (A) verhindernden Zusatzstoffs, ausgewählt aus hydrophoben Wachsen, hydrophoben
weichwachsartigen Stoffen oder hydrophoben Ölen. Die Produkte sind vorzugsweise wasserfrei, können aber auch geringe Mengen Wasser bis maximal 15 Gew.%, vorzugsweise maximal 10 Gew.% enthalten.

Die Auswaschbarkeit des Produktes ist trotz der hydrophoben Natur des Zusatzstoffes gut, die übrigen Anwendungseigenschaften werden nicht nachteilig beeinflusst, unerwünschte Kristallisationen des wachsartigen Polyethylenglykols in der Produktmasse werden reduziert und die Konsistenz zeichnet sich durch eine größere Homogenität aus.

Die wachsartige Konsistenz ist vorzugsweise dadurch gekennzeichnet, dass die Nadelpenetrationszahl (Maßeinheit 0,1 mm, Prüfgewicht 100 g, Prüfdauer 5 s, Prüftemperatur 25°C; nach DIN 51 579) größer oder gleich 10, besonders bevorzugt größer oder gleich 20 ist und dass der Erstarrungspunkt des Produktes vorzugsweise größer oder gleich 30°C ist und besonders bevorzugt im Bereich von 40 bis 55°C liegt.

### Wachsartige Polyethylenglykole

Die wachsartigen Polyethylenglykole (A) sind bei Raumtemperatur (25°C) wachsartig fest, d.h. die Erstarrungstemperatur liegt oberhalb 25°C, vorzugsweise im Bereich von 35 bis 60 °C, besonders bevorzugt von 45 bis 60°C. Sie werden in Konzentrationen von 25 bis 60 Gew.%, besonders bevorzugt von 30 bis 50 Gew.% eingesetzt. Das Molekulargewicht liegt vorzugsweise im Bereich von 950 bis 30000, besonders bevorzugt von 1800 bis 5000 g/mol, insbesondere von 2500 bis 3500 g/mol. Polyethylenglykole besitzen die allgemeine Formel H(OCH₂CH₂)ₙOH. Geeignet sind z.B. solche mit n = 20 bis 220, vorzugsweise mit n = 40 bis 100, besonders bevorzugt von 50 bis 80. Geeignete Polyethylenglykole haben z.B. die INCI-Bezeichnungen PEG-20, PEG-32, PEG-40, PEG-45, PEG-55, PEG-60, PEG-75, PEG-90 oder PEG-100. Besonders bevorzugt sind PEG-60 und PEG-75. Handelsprodukte weisen in der Regel eine Molekulargewichtsverteilung auf. Geeignet sind z.B. Polyglykol 3000 mit einem Molekulargewicht von 2700 bis 3000 oder Polyglykol 4000 der Firma Clariant mit einem Molekulargewicht von 3700 bis 4500.

### Zusätzliches flüssiges Polyethylenglykol

In einer besonderen Ausführungsform ist zur Verbesserung der Produktleistungen insbesondere hinsichtlich einer besseren Verteilbarkeit und Einarbeitbarkeit im Haar zusätzlich ein weiteres Polyethylenglykol enthalten. Dieses ist bei Raumtemperatur (25°C) flüssig. Es wird vorzugsweise in einer Menge von mindestens 15 Gew.%, besonders bevorzugt von 30 bis 70 Gew.%, insbesondere von 40 bis 60 Gew.% eingesetzt. Das Gewichtsverhältnis des festen PEG (A) zum zusätzlichen, flüssigen PEG (C) beträgt vorzugsweise von 0,5 bis 1,25, besonders bevorzugt von 0,6 bis 1. Das Molekulargewicht liegt vorzugsweise im Bereich von 100 bis 700, besonders bevorzugt von 350 bis 700, insbesondere von 500 bis 650 g/mol. Geeignete flüssige PEG sind solche der Formel H(OCH₂CH₂)ₙOH mit n = 4 bis 14, vorzugsweise mit n = 8 bis 12. Geeignete PEG haben die INCI-Bezeichnungen PEG-4, PEG-6, PEG-7, PEG-8, PEG-9, PEG-10, PEG-12 und PEG-14, wobei PEG-10 und PEG-12 besonders bevorzugt sind. Geeignete Handelsprodukte sind z.B. Polyglykol 400 mit einem Molekulargewicht von 380 bis 420 oder Polyglykol 600 der Firma Clariant mit einem Molekulargewicht von 570 bis 630.

### Hydrophobe Zusatzstoffe

Der die Kristallisation des Polyethylenglykols (A) verhindernde Zusatzstoff (B) wird in einer Menge von größer 5 Gew.% bis 30 Gew.%, besonders bevorzugt von 10 bis 20 Gew.% eingesetzt. Geeignete Zusatzstoffe sind hydrophobe Wachse, hydrophobe weichwachsartige Stoffe oder hydrophobe Öle.

Geeignete hydrophobe Wachse sind z.B. tierische, pflanzliche, mineralische und synthetische Wachse, mikrokristalline Wachse, makrokristalline Wachse, feste Paraffine, Ozokerit, Montanwachs, Fischer-Tropsch-Wachse, Polyolefinwachse z.B. Polybuten, Bienenwachs, Wollwachs (Lanolin) und dessen Derivate wie z.B. Wollwachsalkohole, Candelillawachs, Carnaubawachs, Japanwachs, gehärtete Fette, Fettsäureester und Fettsäureglyceride mit einem Erstarrungspunkt von jeweils oberhalb 40°C, Polyethylenwachse und Silikonwachse. Die Wachse oder wachsartigen Stoffe haben einen Erstarrungspunkt oberhalb 40°C, vorzugsweise oberhalb 55 °C. Geeignete hydrophobe weichwachsartige Stoffe sind z.B. halbfeste Paraffine. Die Erstarrungspunkte liegen in der Regel im Bereich von ca. 25°C bis 40°C. Besonders bevorzugt als hydrophobe Zusatzstoffe sind Produkte mit der INCI-Bezeichnung Petrolatum, z.B. Vaseline. Hierbei handelt es sich um eine halbfeste Mischung von aus Petroleum gewonnenen Kohlenwasserstoffen.

Geeignete hydrophobe Öle haben einen Schmelzpunkt von unterhalb 25°C und einen Siedepunkt von vorzugsweise über 250 °C, insbesondere über 300 °C. Hierfür kann prinzipiell jedes dem Fachmann allgemein bekannte Öl eingesetzt werden. In Frage kommen pflanzliche oder tierische Öle, Mineralöle (Paraffinum liquidum), Silikonöle oder deren Mischungen. Geeignete Silikonöle sind Polydimethylsiloxane, phenylierte Silikone, Polyphenylmethylsiloxane, Phenyltrimethicone, Poly(C1-C20)-alkylsiloxane, Alkylmethylsiloxane. Geeignet sind weiterhin Kohlenwasserstofföle, z.B. Paraffin- oder Isoparaffinöle, Squalan, Öle aus Fettsäuren und Polyolen, insbesondere Triglyceride. Geeignete pflanzliche Öle sind z.B. Sonnenblumenöl, Kokosöl, Rizinusöl, Lanolinöl, Jojobaöl, Maisöl, Sojaöl.

### Emulgatoren

In einer bevorzugten Ausführungsform enthält das erfindungsgemäße Haarwachsprodukt zusätzlich mindestens einen Emulgator. Die Emulgatoren sind vorzugsweise in einer Menge von 0,1 bis 20 Gew.%, besonders bevorzugt von 0,2 bis 10 Gew.% enthalten. Die Emulgatoren können nichtionisch, anionisch, kationisch oder zwitterionisch sein, wobei nichtionische Emulgatoren bevorzugt werden. Geeignet sind z.B.
- Ethoxylierungsprodukte von Fettalkoholen, Fettsäuren, Fettsäureglyceriden oder Alkylphenolen, insbesondere Anlagerungsprodukte von 2 bis 30 mol Ethylenoxid und/oder 1 bis 5 mol Propylenoxid an C8- bis C22-Fettalkohole, an C12- bis C22-Fettsäuren oder an Alkylphenole mit 8 bis 15 C-Atomen in der Alkylgruppe,
- C12- bis C22-Fettsäuremono- und -diester von Anlagerungsprodukten von 1 bis 30 mol Ethylenoxid an Glycerin,
- Anlagerungsprodukte von 5 bis 60 mol Ethylenoxid an Rizinusöl oder an gehärtetes (hydriertes) Rizinusöl.
- Mono-, Di- und/oder Triester der Phosphorsäure mit Anlagerungsprodukten von 2 bis 30 mol Ethylenoxid an C8-bis C22-Fettalkohole
- Fettsäurezuckerester, insbesondere Ester aus Saccharose und ein oder zwei C8- bis C22-Fettsäuren, INCI: Sucrose Cocoate, Sucrose Dilaurate, Sucrose Distearate, Sucrose Laurate, Sucrose Myristate, Sucrose Oleate, Sucrose Palmitate, Sucrose Ricinoleate, Sucrose Stearate
- Polyglycerylfettsäureester, insbesondere aus ein, zwei oder mehreren C8- bis C22-Fettsäuren und Polyglycerin mit vorzugsweise 2 bis 20 Glyceryleinheiten.

In einer besonders bevorzugten Ausführungsform haben die Emulgatoren wachsartige Konsistenz und einen Tropfpunkt oberhalb 25°C.

### Pigmente

In einer bevorzugten Ausführungsform enthält das erfindungsgemäße Haarwachsprodukt zusätzlich mindestens ein Pigment. Hierbei kann es sich um farbige Pigmente handeln, welche der Produktmasse oder dem Haar Farbeffekte verleihen oder es kann sich um Glanzeffektpigmente handeln, welche der Produktmasse oder dem Haar Glanzeffekte verleihen. Die Farb- oder Glanzeffekte am Haar sind vorzugsweise temporär, d.h. sie halten bis zur nächsten Haarwäsche und können durch Waschen der Haare mit üblichen Shampoos wieder entfernt werden. Die Pigmente liegen in der Produktmasse in ungelöster Form vor und können in einer Menge von 0,01 bis 25 Gew.%, besonders bevorzugt von 5 bis 15 Gew.% enthalten sein.

Bei den Pigmenten handelt es sich vorzugsweise nicht um Nano- sonder um Mikropigmente. Die bevorzugte Teilchengröße beträgt 1 bis 200 µm, insbesondere 3 bis 150 µm, besonders bevorzugt 10 bis 100 µm.

Die Pigmente sind im Anwendungsmedium praktisch unlösliche Farbmittel und können anorganisch oder organisch sein. Auch anorganisch-organische Mischpigmente sind möglich. Bevorzugt sind anorganische Pigmente. Der Vorteil der anorganischen Pigmente ist deren ausgezeichnete Licht-, Wetter- und Temperaturbeständigkeit. Die anorganischen Pigmente können natürlichen Ursprungs sein, beispielsweise hergestellt aus Kreide, Ocker, Umbra, Grünerde, gebranntem Terra di Siena oder Graphit. Bei den Pigmenten kann es sich um Weißpigmente wie z.B. Titandioxid oder Zinkoxid, um Schwarzpigmente wie z.B. Eisenoxidschwarz, Buntpigmente wie z.B. Ultramarin oder Eisenoxidrot, um Glanzpigmente, Metalleffekt-Pigmente, Perlglanzpigmente sowie um Fluoreszenz- oder Phosphoreszenzpigmente handeln, wobei vorzugsweise mindestens ein Pigment ein farbiges, nicht-weißes Pigment ist. Geeignet sind Metalloxide, -hydroxide und -oxidhydrate, Mischphasenpigmente, schwefelhaltige Silicate, Metallsulfide, komplexe Metallcyanide, Metallsulfate, -chromate und -molybdate sowie die Metalle selbst (Bronzepigmente). Geeignet sind insbesondere Titandioxid (CI 77891), schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510), Carmine (Cochineal).

Besonders bevorzugt sind Perlglanz- und Farbpigmente auf Mica- bzw. Glimmerbasis welche mit einem Metalloxid oder einem Metalloxychlorid wie Titandioxid oder Wismutoxychlorid sowie gegebenenfalls weiteren farbgebenden Stoffen wie Eisenoxiden, Eisenblau, Ultramarine, Carmine etc. beschichtet sind und wobei die Farbe durch Variation der Schichtdicke bestimmt sein kann. Derartige Pigmente werden beispielsweise unter den Handelsbezeichnungen Rona® , Colorona®, Dichrona® und Timiron® von der Firma Merck, Deutschland vertrieben.

Organische Pigmente sind beispielsweise die natürlichen Pigmente Sepia, Gummigutt, Knochenkohle, Kasseler Braun, Indigo, Chlorophyll und andere Pflanzenpigmente. Synthetische organische Pigmente sind beispielsweise Azo-Pigmente, Anthrachinoide, Indigoide, Dioxazin-, Chinacridon-, Phtalocyanin-, Isoindolinon-, Perylen- und Perinon-, Metallkomplex-, Alkaliblau- und Diketopyrrolopyrrol-Pigmente.

### Optionale Zusatzstoffe

Zusätzlich zu den vorstehend genannten Inhaltsstoffen kann die Zusammensetzung weitere Zusatzstoffe enthalten:
- Lösungsmittel wie Wasser oder ein- oder mehrwertige C1-bis C4-Alkohole, insbesondere Ethanol, Isopropanol, Glycerine oder Glykole in einer Menge bis zu 15 Gew.%, vorzugsweise 0,1 bis 8 Gew.%
- Gelöste kosmetische Farbstoffe in einer Menge bis zu 6 Gew.%, vorzugsweise 0,1 bis 4 Gew.%
- Parfüm- und Duftstoffe in einer Menge von bis zu 2 Gew.%, vorzugsweise 0,01 bis 1 Gew.%
- Konservierungsmittel in einer Menge von bis zu 1 Gew.%, vorzugsweise 0,01 bis 0,5 Gew.%, insbesondere Parahydroxybenzoesäureester, Benzoesäure, Salicylsäure, Sorbinsäure, Mandelsäure, Polyhexamethylen-biguanid-hydrochlorid oder Isothiazolinonderivate
- Filmbildende Polymere wie z.B. Polyvinylpyrrolidon oder Vinylpyrrolidon/Vinylacetat Copolymere in einer Menge von bis zu 5 Gew.%, vorzugsweise 0,1 bis 4 Gew.%
- Haarpflegende Zusätze wie z.B. Betain in einer Menge von bis zu 5 Gew.%, vorzugsweise 0,01 bis 4 Gew.%.

### Bevorzugte Ausführungsform

Eine bevorzugte Ausführungsform ist ein Haarwachsprodukt mit einem Gehalt an
(A) 30 bis 60 Gew.% an mindestens einem Polyethylenglykol mit einem Molekulargewicht im Bereich von 950 bis 30000,
(B) 10 bis 30 Gew.% mindestens eines hydrophoben Wachses, hydrophoben weichwachsartigen Stoffes oder hydrophoben Öls,
(C) 30 bis 60 Gew.% an mindestens einem Polyethylenglykol mit einem Molekulargewicht im Bereich von 200 bis 700,
(D) 0 bis 15 Gew.% mindestens eines Emulgators,
(E) 0 bis 15 Gew.% Wasser,
(F) 0 bis 10 Gew.% mindestens eines Zusatzstoffes, ausgewählt aus ein- oder mehrwertigen C1-C5-Alkoholen, Parfüm- und Duftstoffen, Farbstoffen, Konservierungsmitteln, Lichtschutzstoffen, filmbildenden Polymeren, haarpflegenden Wirkstoffen und Perlglanzpigmenten,
wobei die Nadelpenetrationszahl des Produktes bei 25°C größer oder gleich 10 und der Erstarrungspunkt größer oder gleich 30°C ist.

### Herstellverfahren

Erfindungsgemäße Haarwachsprodukte können hergestellt werden, indem die festen Wachskomponenten aufgeschmolzen und vermischt werden. Anschließend wird abgekühlt und kurz vor dem Erstarren werden gegebenenfalls leichtflüchtige Zusatzstoffe zugegeben und vermischt. Die noch fließfähige Masse wird vor dem Erstarren in die gewünschte Verpackung, z.B. Kunststofftiegel abgefüllt.

### Anwendungsverfahren

Die Applikation des erfindungsgemäßen Haarwachsproduktes ist sehr einfach. Es wird bevorzugt auf trockenem Haar angewendet. Eine Menge, die in Abhängigkeit der Haarlänge varriieren kann, z.B. von ungefähr Erbsengröße bis ungefähr Haselnussgröße wird mit den Fingern entnommen. Das Wachs wird in den Handflächen verrieben und durch die Handwärme und durch die Verreibung erfolgende Scherung aufgeschmolzen oder zumindest stark erweicht. Im erweichten oder mehr oder weniger flüssigen Zustand wird das Wachs in das Haar eingearbeitet und die gewünschte Frisur erstellt. Es können auch separate Haarsträhnen einzeln behandelt werden, um diese zu akzentuieren. Im Haar erhärtet das Wachs und die gestaltete Frisur erhält Stabilität, Glanz, Texture und Halt.

Das erfindungsgemäße Haarwachsprodukt ermöglicht aufgrund der wachsartigen Konsistenz und seiner kohäsiven Eigenschaften eine individuelle Frisurengestaltung sowie die gezielte Behandlung einzelner Haarsträhnen. Die Produktmasse ist leicht auf dem Haar verteilbar. Das behandelte Haar zeichnet sich durch einen ausgeprägten Glanz sowie durch eine hohe Formstabilität der erstellten Frisur aus. Das Haar wird dabei nicht übermäßig belastet und die Produktmasse ist leicht auswaschbar. Die Produktmasse neigt auch über längere Zeiträume hinweg nicht zu partiellen Kristallisierungen, bildet daher keine Körnchen und sieht homogen aus.

Die nachfolgenden Beispiele sollen den Gegenstand der Erfindung näher erläutern.

### Beispiele

### Beispiel 1

| | A | B | C |
|---|---|---|---|
| PEG-60 | 40 g | 40 g | 40 g |
| PEG-12 | 50 g | 50 g | 25 g |
| PEG-20 | - | - | 25 g |
| Petrolatum | 10 g | - | - |
| PEG-25 Hydrogenated Castor Oil | 0,5 g | 0,5 g | 0,5 g |
| Wasser | - | Ad 100 g | Ad 100 g |

Die Inhaltstoffe der Zusammensetzungen werden bei erhöhter Temperatur aufgeschmolzen, miteinander vermischt, in einen Kunststofftiegel abgefüllt und abkühlen gelassen.

Die erfindungsgemäße Zusammenstzung A wurde hinsichtlich ihrer Konsistenz mit den nicht erfindungsgemäßen Vergleichszusammensetzungen B und C verglichen. Zusammensetzung A ist deutlich homogener, weniger spöde, weniger körnig, läßt sich besser der Verpackung entnehmen, ist besser mit den Händen verteilbar und erzeugt nach der Anwendung auf dem Haar mehr Glanz als Zusammensetzungen B und C.

### Beispiel 2

| | |
|---|---|
| 40-60 g | PEG-12 |
| 30-50 g | PEG-60 |
| 10-20 g | Petrolatum |
| q.s. | Emulgator |
| q.s. | Parfüm |

### Beispiel 3

| | |
|---|---|
| 40-60 g | PEG-12 |
| 30-50 g | PEG-60 |
| 0-20 g | Petrolatum |
| 5-10 g | Carnaubawachs oder Cera alba |
| q.s. | Emulgator |
| q.s. | Parfüm |

### Beispiel 4

| | |
|---|---|
| 38 g | PEG-60 |
| 50 g | PEG-12 |
| 10 g | Petrolatum |
| 0,5 g | Timiron® Super Silver (Mica/Titandioxid Pigment) |
| 0,5 g | PEG-25 Hydrogenated Castor Oil |
| 0,1 g | D-Panthenol |
| q.s. | Parfüm |
| ad 100 g | Wasser |

### Beispiel 5

| | |
|---|---|
| 38 g | PEG-60 |
| 25 g | PEG-12 |
| 25 g | PEG-20 |
| 10 g | Petrolatum |
| 0,5 g | Timiron® Super Silver (Mica/Titandioxid Pigment) |
| 0,5 g | PEG-25 Hydrogenated Castor Oil |
| 0,1 g | D-Panthenol |
| q.s. | Parfüm |
| ad 100 g | Wasser |

### Beispiel 6

| | |
|---|---|
| 33 g | PEG-60 |
| 45 g | PEG-12 |
| 20 g | Lanolin |
| 0,5 g | PEG-25 Hydrogenated Castor Oil |
| q.s. | Parfüm |

## Patentansprüche

1. Haarwachsprodukt mit wachsartiger Konsistenz zur Behandlung oder Erstellung der menschlichen Frisur mit einem Gehalt an
(A) 25 bis 60 Gew.% mindestens eines bei Raumtemperatur, d.h. bei 25°C wachsartig festen Polyethylenglykols und
(B) größer 5 bis 30 Gew.% mindestens eines die Kristallisation des Polyethylenglykols (A) verhindernden Zusatzstoffs, ausgewählt aus hydrophoben Wachsen, hydrophoben weichwachsartigen Stoffen und hydrophoben Ölen.

2. Haarwachsprodukt nach Anspruch 1, **dadurch gekennzeichnet, dass** es wasserfrei ist oder maximal 15 Gew.% Wasser enthält.

3. Haarwachsprodukt nach einem der vorhergehenden Ansprüche, **dadurch gekennzeichnet, dass** die Nadelpenetrationszahl bei 25°C größer oder gleich 10 ist, gemessen in der Maßeinheit 0,1 mm mit einem Prüfgewicht von 100 g und einer Prüfdauer von 5 s nach DIN 51 579 und dass der Erstarrungspunkt größer oder gleich 30°C ist.

4. Haarwachsprodukt nach einem der vorhergehenden Ansprüche, **dadurch gekennzeichnet, dass** das Polyethylenglykol (A) ein Molekulargewicht aufweist im Bereich von 950 bis 30000.

5. Haarwachsprodukt nach einem der vorhergehenden Ansprüche, **dadurch gekennzeichnet, dass** zusätzlich ein weiteres, bei Raumtemperatur (25°C) flüssiges Polyethylenglykol (C) mit einem Molekulargewicht im Bereich von 100 bis 700 in einer Menge von 30 bis 70 Gew.% enthalten ist.

6. Haarwachsprodukt nach Anspruch 5, **dadurch gekennzeichnet, dass** das Gewichtsverhältnis des wachsartig festen Polyethylenglykols (A) zum flüssigen Polyethylenglykol (C) von 0,5 bis 1,25 beträgt.

7. Haarwachsprodukt nach einem der vorhergehenden Ansprüche, **dadurch gekennzeichnet, dass** der hydrophobe Zusatzstoff (B) Petrolatum ist.

8. Haarwachsprodukt nach einem der vorhergehenden Ansprüche, **dadurch gekennzeichnet, dass** zusätzlich ein Emulgator (D) in einer Menge von 0,1 bis 20 Gew.% enthalten ist.

9. Wachsprodukt nach Anspruch 8, **dadurch gekennzeichnet, dass** der Emulgator (D) ausgewählt ist aus
a) ethoxylierten Fettalkoholen, ethoxylierten Fettsäuren, ethoxylierten Fettsäureglyceriden oder ethoxylierten Alkylphenolen, insbesondere Anlagerungsprodukte von 2 bis 30 mol Ethylenoxid und/oder 1 bis 5 mol Propylenoxid an C8- bis C22-Fettalkohole, an C12- bis C22-Fettsäuren oder an Alkylphenole mit 8 bis 15 C-Atomen in der Alkylgruppe,
b) C12- bis C22-Fettsäuremono- und -diestern von Anlagerungsprodukten von 1 bis 30 mol Ethylenoxid an Glycerin,
c) Anlagerungsprodukten von 5 bis 60 mol Ethylenoxid an Rizinusöl oder an gehärtetes (hydriertes) Rizinusöl,
d) Mono-, Di- und Triestern der Phosphorsäure mit Anlagerungsprodukten von 2 bis 30 mol Ethylenoxid an C8- bis C22-Fettalkohole,
e) Fettsäurezuckerestern, insbesondere Ester aus Saccharose und ein oder zwei C8- bis C22-Fettsäuren,
f) Polyglycerylfettsäureestern, insbesondere aus ein, zwei oder mehreren C8- bis C22-Fettsäuren und Polyglycerin mit vorzugsweise 2 bis 20 Glyceryleinheiten,
oder aus Gemischen dieser Emulgatoren.

10. Haarwachsprodukt nach einem der vorhergehenden Ansprüche, **dadurch gekennzeichnet, dass** es zusätzlich mindestens ein Pigment enthält.

11. Haarwachsprodukt nach einem der vorhergehenden Ansprüche mit einem Gehalt an
(A) 30 bis 60 Gew.% an mindestens einem Polyethylenglykol mit einem Molekulargewicht im Bereich von 950 bis 30000,
(B) 10 bis 30 Gew.% mindestens eines hydrophoben Wachses, hydrophoben weichwachsartigen Stoffes oder hydrophoben Öls,
(C) 30 bis 60 Gew.% an mindestens einem Polyethylenglykol mit einem Molekulargewicht im Bereich von 200 bis 700
(D) 0 bis 15 Gew.% mindestens eines Emulgators und
(E) 0 bis 15 Gew.% Wasser,
(F) 0 bis 10 Gew.% mindestens eines Zusatzstoffes, ausgewählt aus ein- oder mehrwertigen C1-C5-Alkoholen, Parfüm- und Duftstoffen, Farbstoffen, Konservierungsmitteln, Lichtschutzstoffen, filmbildenden Polymeren, haarpflegenden Wirkstoffen und Perlglanzpigmenten,
wobei die Nadelpenetrationszahl des Produktes bei 25°C größer oder gleich 10 und der Erstarrungspunkt größer oder gleich 30°C ist.

12. Verwendung eines Haarwachsproduktes mit wachsartiger Konsistenz enthaltend
(A) mehr als 20 Gew.% mindestens eines bei Raumtemperatur, d.h. bei 25°C wachsartig festen Polyethylenglykols und
(B) größer 5 Gew.% an mindestens einem die Kristallisation des Polyethylenglykols (A) verhindernden Zusatzstoffs, ausgewählt aus hydrophoben Wachsen, hydrophoben weichwachsartigen Stoffen und hydrophoben Ölen
zur Erzeugung von Halt, Festigung oder Stand der menschlichen Frisur.

## Claims

1. Hair wax product with waxy consistency for the treatment or creation of the human hairstyle, with a content of
(A) 25 to 60% by weight of at least one polyethylene glycol which is waxy solid at room temperature, i.e. at 25°C, and
(B) greater than 5 to 30% by weight of at least one additive which prevents crystallization of the polyethylene glycol (A), chosen from hydrophobic waxes, hydrophobic soft-waxy substances and hydrophobic oils.

2. Hair wax product according to Claim 1, **characterized in that** it is anhydrous or comprises a maximum of 15% by weight of water.

3. Hair wax product according to one of the preceding claims, **characterized in that** the needle penetration number at 25°C is greater than or equal to 10, measured in the measurement unit 0.1 mm with a test weight of 100 g and a test time of 5 s in accordance with DIN 51 579, and that the solidification point is greater than or equal to 30°C.

4. Hair wax product according to one of the preceding claims, **characterized in that** the polyethylene glycol (A) has a molecular weight in the range from 950 to 30 000.

5. Hair wax product according to one of the preceding claims, **characterized in that** a further polyethylene glycol (C) which is liquid at room temperature (25°C) and has a molecular weight in the range from 100 to 700 is additionally present in an amount of from 30 to 70% by weight.

6. Hair wax product according to Claim 5, **characterized in that** the weight ratio of the waxy solid polyethylene glycol (A) to the liquid polyethylene glycol (C) is from 0.5 to 1.25.

7. Hair wax product according to one of the preceding claims, **characterized in that** the hydrophobic additive (B) is petrolatum.

8. Hair wax product according to one of the preceding claims, **characterized in that** an emulsifier (D) is additionally present in an amount of from 0.1 to 20% by weight.

9. Wax product according to Claim 8, **characterized in that** the emulsifier (D) is chosen from
a) ethoxylated fatty alcohols, ethoxylated fatty acids, ethoxylated fatty acid glycerides or ethoxylated alkylphenols, in particular addition products of from 2 to 30 mol of ethylene oxide and/or 1 to 5 mol of propylene oxide onto C8-C22-fatty alcohols, onto C12-C22-fatty acids or onto alkylphenols having 8 to 15 carbon atoms in the alkyl group,
b) C12-C22-fatty acid mono- and diesters of addition products of from 1 to 30 mol of ethylene oxide onto glycerol,
c) addition products of from 5 to 60 mol of ethylene oxide onto castor oil or onto hardened (hydrogenated) castor oil,
d) mono- di- and triesters of phosphoric acid with addition products of from 2 to 30 mol of ethylene oxide onto C8-C22-fatty alcohols,
e) fatty acid sugar esters, in particular esters of sucrose and one or two C8-C22-fatty acids,
f) polyglyceryl fatty acid esters, in particular of one, two or more C8-C22-fatty acids and polyglycerol with preferably 2 to 20 glyceryl units,
or of mixtures of these emulsifiers.

10. Hair wax product according to one of the preceding claims, **characterized in that** it additionally comprises at least one pigment.

11. Hair wax product according to one of the preceding claims with a content of
(A) 30 to 60% by weight of at least one polyethylene glycol with a molecular weight in the range from 950 to 30 000,
(B) 10 to 30% by weight of at least one hydrophobic wax, hydrophobic soft-waxy substance or hydrophobic oil,
(C) 30 to 60% by weight of at least one polyethylene glycol with a molecular weight in the range from 200 to 700
(D) 0 to 15% by weight of at least one emulsifier and
(E) 0 to 15% by weight of water,
(F) 0 to 10% by weight of at least one additive, chosen from mono- or polyhydric C1-C5-alcohols, perfumes and fragrances, dyes, preservatives, light protection substances, film-forming polymers, haircare active ingredients and pearlescent pigments, where the needle penetration number of the product at 25°C is greater than or equal to 10 and the solidification point is greater than or equal to 30°C.

12. Use of a hair wax product with waxy consistency comprising
(A) more than 20% by weight of at least one polyethylene glycol which is waxy solid at room temperature, i.e. at 25°C, and
(B) more than 5% by weight of at least one additive which prevents crystallization of the polyethylene glycol (A), chosen from hydrophobic waxes, hydrophobic soft-waxy substances and hydrophobic oils
for producing hold, setting or stand of the human hairstyle.

## Revendications

1. Composition capillaire cireuse à consistance cireuse, pour le traitement ou l'élaboration de la coiffure humaine, contenant
(A) 25 à 60 % en poids d'au moins un polyéthylèneglycol solide cireux à la température ambiante, c'est-à-dire à 25°C et
(B) plus de 5 à 30 % en poids d'au moins un additif empêchant la cristallisation du polyéthylèneglycol (A), choisi parmi des cires hydrophobes, des substances hydrophobes de type cire molle et des huiles hydrophobes.

2. Composition capillaire cireuse selon la revendication 1, **caractérisée en ce qu'**elle est pratiquement anhydre ou contient au maximum 15 % en poids d'eau.

3. Composition capillaire cireuse selon l'une quelconque des revendications précédentes, **caractérisée en ce que** l'indice de pénétration d'aiguille à 25°C est égal ou supérieur à 10, mesuré dans l'unité de mesure 0,1 mm avec un poids d'essai de 100 g et une durée d'essai de 5 secondes selon DIN 51 579 et **en ce que** le point de solidification est égal ou supérieur à 30°C.

4. Composition capillaire cireuse selon l'une quelconque des revendications précédentes, **caractérisée en ce que** le polyéthylèneglycol (A) présente une masse moléculaire dans la plage allant de 950 à 30 000.

5. Composition capillaire cireuse selon l'une quelconque des revendications précédentes, **caractérisée en ce qu'**additionnellement un autre polyéthylèneglycol (C) liquide à la température ambiante (25°C), ayant une masse moléculaire dans la plage de 100 à 700, est contenu en une quantité de 30 à 70 % en poids.

6. Composition capillaire cireuse selon la revendication 5, **caractérisée en ce que** le rapport pondéral du polyéthylèneglycol (A) solide cireux au polyéthylèneglycol (C) liquide va de 0,5 à 1,25.

7. Composition capillaire cireuse selon l'une quelconque des revendications précédentes, **caractérisée en ce que** l'additif hydrophobe (B) est la vaseline.

8. Composition capillaire cireuse selon l'une quelconque des revendications précédentes, **caractérisée en ce qu'**additionnellement un émulsifiant (D) est contenu en une quantité de 0,1 à 20 % en poids.

9. Composition cireuse selon la revendication 8, **caractérisée en ce que** l'émulsifiant (D) est choisi parmi
a) des alcools gras éthoxylés, des acides gras éthoxylés, des glycérides d'acides gras éthoxylés ou des alkylphénols éthoxylés, en particulier des produits de fixation par addition de 2 à 30 moles oxyde d'éthylène et/ou de 1 à 5 moles d'oxyde de propylène sur des alcools gras en C₈-C₂₂, sur des acides gras en C₁₂-C₂₂ ou sur des alkylphénols ayant de 8 à 15 atomes de carbone dans le groupe alkyle,
b) des mono- et diesters d'acides gras en C₁₂-C₂₂ avec des produits de fixation par addition de 1 à 30 moles d'oxyde d'éthylène sur le glycérol,
c) des produits de fixation par addition de 5 à 60 moles d'oxyde d'éthylène sur l'huile de ricin ou sur de l'huile de ricin durcie (hydrogénée),
d) des mono-, di- et triesters de l'acide phosphorique avec des produits de fixation par addition de 2 à 30 moles d'oxyde d'éthylène sur des alcools gras en C₈-C₂₂,
e) des esters glycidiques d'acides gras, en particulier des esters de saccharose et d'un ou deux acides gras en C₈-C₂₂,
f) des polyglycérylesters d'acides gras, en particulier d'un, de deux ou de plus de deux acides gras en C₈-C₂₂ et de polyglycérol comportant de préférence de 2 à 20 motifs glycéryle,
ou des mélanges de ces émulsifiants.

10. Composition capillaire cireuse selon l'une quelconque des revendications précédentes, **caractérisée en ce qu'**elle contient additionnellement au moins un pigment.

11. Composition capillaire cireuse selon l'une quelconque des revendications précédentes, contenant
(A) 30 à 60 % en poids d'au moins un polyéthylèneglycol ayant une masse moléculaire dans la plage allant de 950 à 30 000,
(B) 10 à 30 % en poids d'au moins une cire hydrophobe, une substance hydrophobe de type cire molle ou une huile hydrophobe,
(C) 30 à 60 % en poids d'au moins un polyéthylèneglycol ayant une masse moléculaire dans la plage allant de 200 à 700,
(D) 0 à 15 % en poids d'au moins un émulsifiant et
(E) 0 à 15 % en poids d'eau,
(F) 0 à 10 % en poids d'au moins un additif choisi parmi des alcools en C1-C5, des parfums et des substances odorantes, des colorants, des conservateurs, des photo-protecteurs, des polymères filmogènes, des substances actives de soin capillaire et des pigments nacrés, l'indice de pénétration d'aiguille de la composition à 25°C étant égal ou supérieur à 10 et le point de solidification étant égal ou supérieur à 30°C.

12. Utilisation d'une composition capillaire cireuse à consistance cireuse, contenant
(A) plus de 20 % en poids d'au moins un polyéthylèneglycol solide cireux à la température ambiante, c'est-à-dire à 25°C et
(B) plus de 5 % en poids d'au moins un additif empêchant la cristallisation du polyéthylèneglycol (A), choisi parmi des cires hydrophobes, des substances hydrophobes de type cire molle et des huiles hydrophobes
pour l'élaboration, le fixage ou la tenue de la coiffure humaine.
